# EUROPEAN PATENT APPLICATION

(11) **EP 3 156 194 A1**
(43) Date of publication of application: **19.04.2017**
(21) Application number: 15806876.7
(22) Date of filing: 28.04.2015
(51) Int. Cl.: B25J 17/02, A61B 90/00

(54) **MANIPULATOR**

(30) Priority: 12.06.2014 JP 2014121332
(71) Applicant: Olympus Corporation, Tokyo 192-8507 (JP)
(72) Inventor: KISHI, Kosuke, Tokyo 192-8507 (JP)
(74) Representative: Schicker, Silvia
(86) International application number: PCT/JP2015/062779
(87) International publication number: WO 2015/190185

(57) **Abstract**

Provided is a manipulator (1) equipped with a joint mechanism (4) that couples an end effector (3) with a body portion (2) via two linkage members (61 and 62), wherein the joint mechanism (4) is provided with: a first joint (11) that couples the end effector (3) with the linkage member (61) in a mutually pivotable manner; a second joint (12) that couples the linkage members (61 and 62) with each other so as to be pivotable about a second axis (A2); a third joint (13) that couples the linkage member (62) with the body portion (2) so as to be pivotable about a third axis (A3) that is parallel to the axis (A2); and a pair of restraining members (71 and 72) that have constant lengths, that flank the second and third axes (A2 and A3) in the radial direction, and that are disposed in a crossed manner so as to intersect each other just once between the second and third joints (12 and 13).

## Description

### {Technical Field}

The present invention relates to a manipulator.

### {Background Art}

In the related art, there is a known manipulator in which three joints are provided between an elongated body portion and an end effector (for example, see Patent Literature 1). Two pitch joints on the base-end side are double joints that work together to flex in the same direction and that are provided with a pair of rolling members that are in contact with each other in a rolling manner. The pair of rolling members are rotatable about rotation axes that are parallel to each other and are configured so that, when the rolling member on the body-portion side is rotationally driven, the rolling member on the end-effector side is also rotated so as to follow this rotation.

The manipulator of Patent Literature 1 is provided with a mechanism with which a yaw joint on the most distal-end side and the two pitch joints are driven independently of each other. Specifically, a driving wire for driving the yaw joint is connected to the yaw joint. The driving wire passes through a pair of pulleys that are coaxial with the pair of rolling members of the pitch joints, and is threaded around the pair of pulleys in a crossed manner. By doing so, the driving wire is not moved in the longitudinal direction when the pitch joints are moved, and thus, the flexing angle of the yaw joint is not changed.

### {Citation List}

### {Patent Literature}

{PTL 1} Publication of Japanese Patent No. 3912251

### {Summary of Invention}

### {Technical Problem}

However, in the case in which the pair of rolling members that are in contact with each other in a rolling manner, as in Patent Literature 1, are used as means for making the two pitch joints work together, there is a problem in that it is difficult to accurately control the flexing angles of the two pitch joints. In other words, in the case of utilizing mutual engagement of gear teeth provided on the outer circumferential surfaces of the rolling members, the rotation-angle ratio (gear ratio) of the pair of rolling members may become unstable due to chipping, wearing, or skipping of the gear teeth. In addition, it may become difficult to set positions in a desired manner due to looseness caused by backlash between the gear teeth. In the case of utilizing friction between the outer circumferential surfaces of the rolling members instead of the gear teeth, slippage may occur between the outer circumferential surfaces. When errors occur in the flexing-angle ratio of the two pitch joints in this way, the driving wire is moved when the pitch joints are moved, and thus, the independence of driving of the yaw joint and the pitch joints is not maintained.

The present invention has been conceived in light of the above-described circumstances, and an object thereof is to provide a manipulator with which, in a manipulator provided with double joints that work together to flex in the same direction, it is possible to achieve stable operating performance of the double joints.

### {Solution to Problem}

In order to achieve the above-described object, the present invention provides the following solutions.

The present invention provides a manipulator including: an elongated body portion; an end effector that is positioned on a distal-end side of the body portion; and a joint mechanism that couples the body portion with the end effector so as to be mutually pivotable about a flexing axis that intersects a longitudinal axis of the body portion, wherein the joint mechanism is provided with: a first linkage member and a second linkage member, sequentially from the distal-end side; a first joint that couples the end effector with the first linkage member so as to be mutually pivotable about a first flexing axis that intersects the longitudinal axis; a second joint that couples the first linkage member with the second linkage member so as to be mutually pivotable about a second flexing axis that intersects the longitudinal axis; a third joint that couples the second linkage member with the body portion so as to be mutually pivotable about a third flexing axis that is parallel to the second flexing axis; and a pair of restraining members that extend from the first linkage member to the body portion by bridging the second joint and the third joint and that have constant lengths, and wherein the pair of restraining members flank the second flexing axis and the third flexing axis in radial directions, respectively, and are disposed in a crossed manner by intersecting each other just once between the second joint and the third joint.

With the present invention, by driving the three joints that are provided in series between the end effector and the body portion, it is possible to pivot the end effector in two directions with respect to the body portion.

In this case, among the three joints, the second joint and the third joint, which are on the base-end side, work together by being driven by the restraining members.

In other words, because the path lengths of the restraining members provided between the first linkage member and the body portion via the second linkage member are constant, the flexing angle of the second joint and the flexing angle of the third joint that are positioned on either side of the second linkage member are restrained with respect to each other. As a result, of the second joint and the third joint, when one of them is flexed, the other one is also flexed in the same direction, and thus, the second joint and the third joint function as double joints. In this way, by mutually restraining the flexing angles of the second and third joints on the basis of the geometric arrangement of the restraining members, it is possible to achieve stable operating performance of the double joints.

The above-described invention may be provided with a driving wire that extends from the first joint to the body portion and that drives the first joint by being pushed and pulled in longitudinal directions thereof; and a pair of pulleys that are provided in the second joint and the third joint so as to be individually rotatable about the second flexing axis and the third flexing axis, and that are arranged with a gap therebetween, having a size equal to or greater than the diameter of the driving wire, in a radial direction, wherein the driving wire may pass through the gap between the pair of pulleys and may be threaded around outer circumferential surfaces of the pair of pulleys in a crossed manner.

By doing so, when the second joint and the third joint are flexed, the driving wire that is threaded, in a crossed manner, around the pair of pulleys provided at the second joint and the third joint is not displaced in the longitudinal direction. Therefore, it is possible to drive the first joint and the second and the third joints independently of each other.

In the above-described invention, the pair of restraining members may be cylindrical sheaths into which the driving wire is inserted in a longitudinal direction in a movable manner.

By doing so, because very small spaces would be sufficient for providing the restraining members, it is possible to reduce the size of the joint mechanism.

In the above-described invention, the pair of restraining members may be secured to the first linkage member at distal ends thereof and may be secured to the body portion at base ends thereof.

By doing so, it is possible to stabilize the positions of the sheaths.

In the above-described invention, it is preferable that the pair of restraining members have the same size in the length direction thereof.

In the above-described invention, the pair of restraining members may be connected with each other in the body portion, and the above-described invention may be provided with a tensile-force adjusting means that is provided in the body portion, that imparts tensile forces to the restraining members, and that is capable of adjusting the magnitudes of the tensile forces.

By doing so, in the case in which the tensile forces in the restraining members decrease, it is possible to adjust the tensile forces so as to achieve a state in which the tensile forces are high again. In addition, because the pair of restraining members are joined together in the body portion, it is possible to simultaneously and evenly adjust the tensile forces in the pair of restraining members. Furthermore, assembly is facilitated, it is possible to realize a treatment tool having stable quality and in which controllability can be enhanced.

The above-described invention may be provided with a pair of guide members that are centered on the second flexing axis and the third flexing axis, respectively, and that have substantially arc-like outer circumferential surfaces that face each other in the direction of the longitudinal axis, wherein a gap, having a width that is equal to or greater than the diameter of the restraining members, may be provided between the outer circumferential surfaces of the pair of guide members, and the restraining members may pass through the gap and may be threaded, in a crossed manner, around the substantially arc-like outer circumferential surfaces of the pair of guide members.

By doing so, it is possible to further stabilize the positions of the restraining members.

In the above-described invention, it is preferable that the width of the gap be equal to the diameter of the restraining members.

By doing so, because the paths through which the restraining members are provided are uniquely set, the flexing angle of the second joint is uniquely set with respect to the flexing angle of the third joint. By doing so, it is possible to further increase the independence of driving of the first joint and the second and third joints.

In the above-described invention, of the pair of guide members, the one on a distal-end side may be integrally provided with the first linkage member at a base end of the first linkage member, and the other one on a base-end side may be integrally provided with the body portion at a distal end of the body portion.

By doing so, it is possible to achieve a cost reduction by decreasing the number of components and the number of assembly steps.

### {Advantageous Effects of Invention}

The present invention affords an advantage in that, in a manipulator provided with double joints that work together to flex in the same direction, it is possible to achieve stable operating performance of the double joints.

### {Brief Description of Drawings}

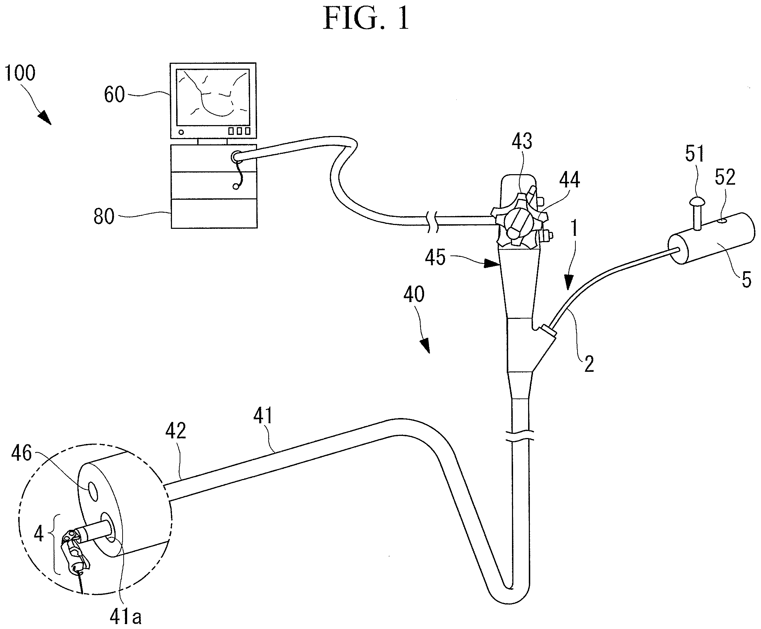

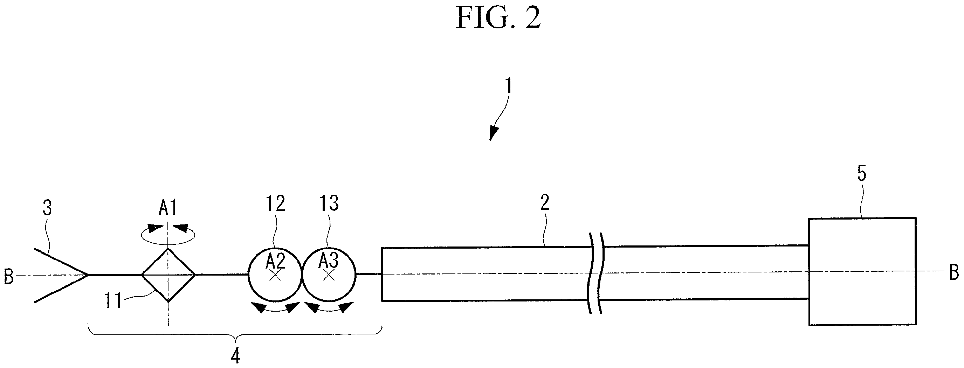

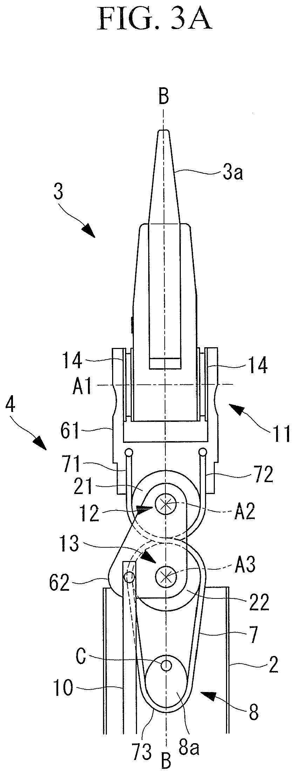

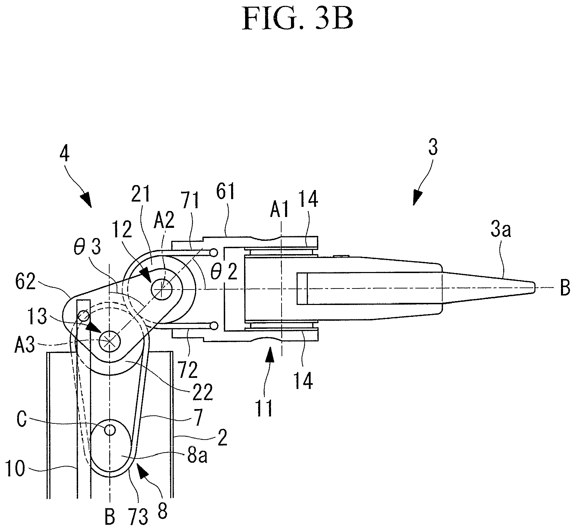

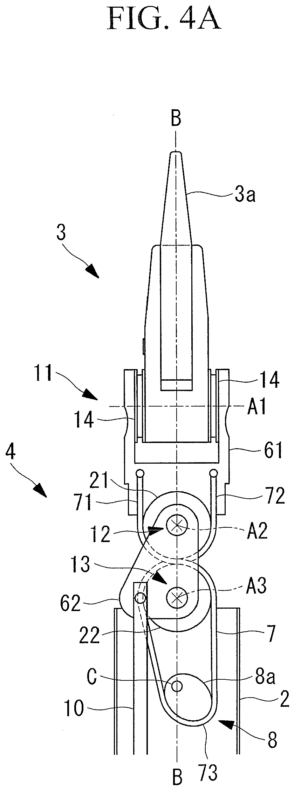

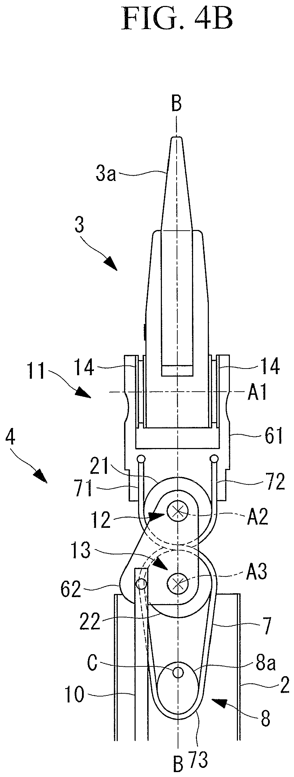

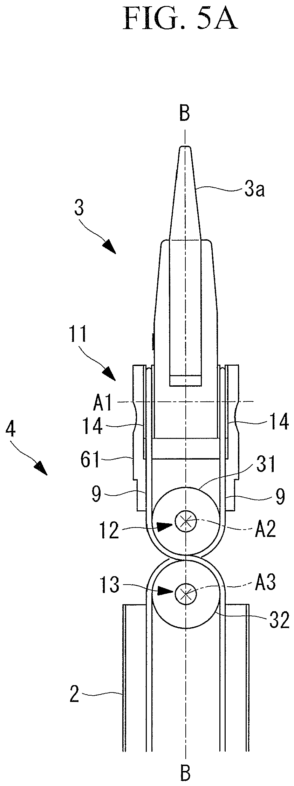

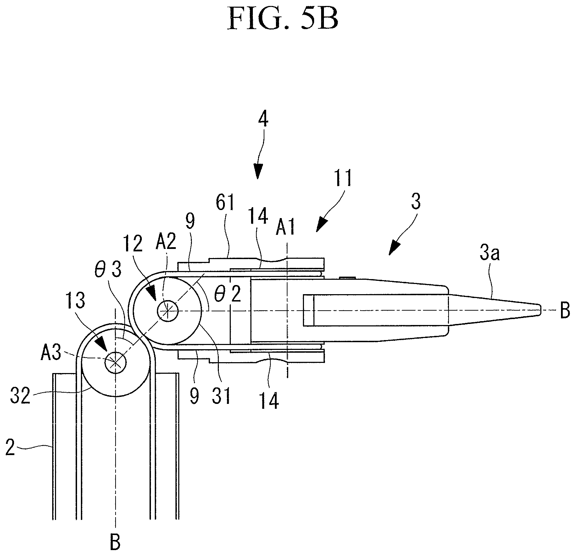

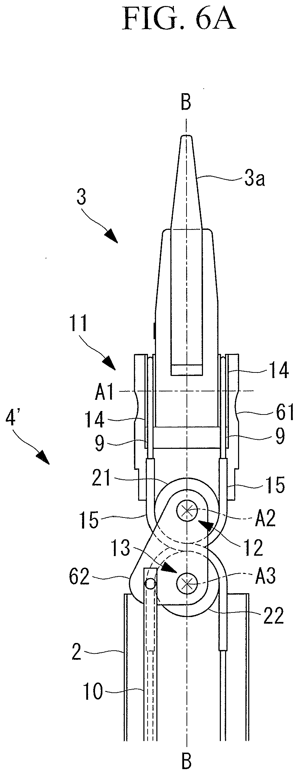

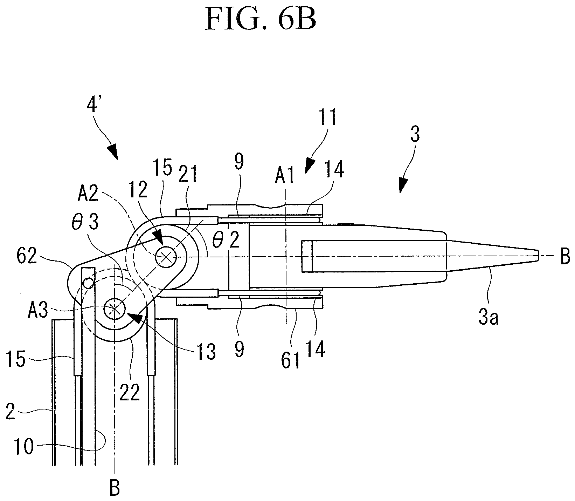

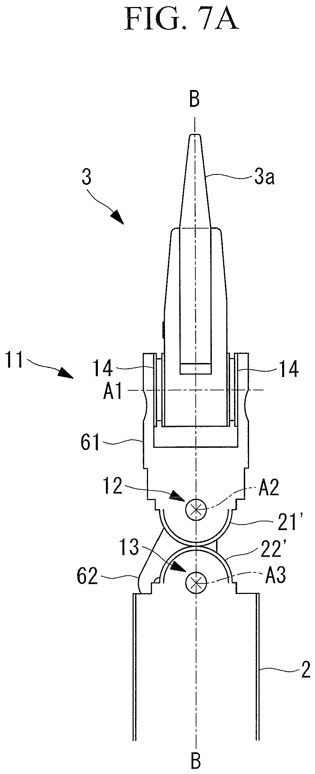

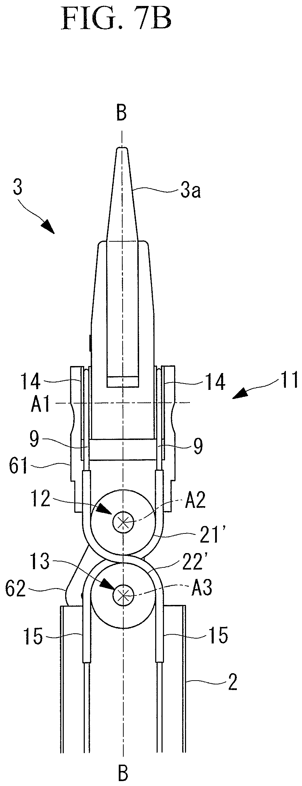

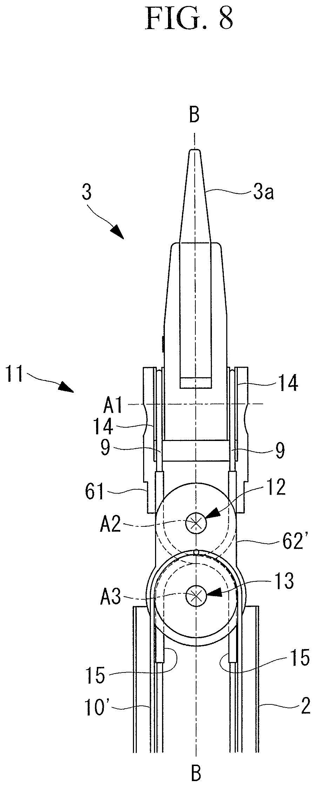

{Fig. 1} Fig. 1 is an overall configuration diagram of a medical system in which manipulators according to first and second embodiments of the present invention are employed.
{Fig. 2} Fig. 2 is a diagram schematically showing the overall configuration of the manipulator according to the first embodiment of the present invention.
{Fig. 3A} Fig. 3A is a configuration diagram for mainly explaining a second joint, a third joint, and a restraining wire provided in a joint mechanism of the manipulator in Fig. 2, showing a state in which the flexing angle of the third joint is 0°.
{Fig. 3B} Fig. 3B is a configuration diagram for mainly explaining the second joint, the third joint, and the restraining wire provided in the joint mechanism of the manipulator in Fig. 2, showing a state in which the flexing angle of the third joint is 45°.
{Fig. 4A} Fig. 4A is a configuration diagram showing the operation of a tensile-force adjusting means in Fig. 3A, showing a state in which the restraining wire is relaxed.
{Fig. 4B} Fig. 4B is a configuration diagram showing the operation of the tensile-force adjusting means in Fig. 3A, showing a state in which the restraining wire is under tension.
{Fig. 5A} Fig. 5A is a configuration diagram for mainly explaining a first joint and driving wires provided in the joint mechanism of the manipulator in Fig. 2, showing a state in which the flexing angle of the third joint is 0°.
{Fig. 5B} Fig. 5B is a configuration diagram for mainly explaining the first joint and the driving wires provided in the joint mechanism of the manipulator in Fig. 2, showing a state in which the flexing angle of the third joint is 45°.
{Fig. 6A} Fig. 6A is a configuration diagram of a joint mechanism provided in a manipulator according to a second embodiment of the present invention, showing a state in which the flexing angle of a third joint is 0°.
{Fig. 6B} Fig. 6B is a configuration diagram of the joint mechanism provided in the manipulator according to the second embodiment of the present invention, showing a state in which the flexing angle of the third joint is 45°.
{Fig. 7A} Fig. 7A is a configuration diagram showing a modification of guide members, showing a state in which driving wires and sheaths are omitted.
{Fig. 7B} Fig. 7B is a configuration diagram showing the guide members in Fig. 7A in a state in which the driving wires and the sheaths are attached.
{Fig. 8} Fig. 8 is a configuration diagram showing a modification provided with a driving wire instead of a driving rod.

### {Description of Embodiments}

First, a medical system 100 in which a manipulator 1 according to an embodiment of the present invention is employed will be described with reference to Fig. 1.

As shown in Fig. 1, the medical system 100 according to this embodiment is provided with: an endoscope 40 that is used by being inserted into a body; a display portion 60 that displays images acquired by using the endoscope 40; and a control portion 80 that controls the entire medical system 100.

The endoscope 40 is provided with: an elongated inserted portion 41 that is inserted into the body of a patient; at least one channel 41a that is formed inside the inserted portion 41 so as to pass therethrough in the longitudinal direction; a bending portion 42 that is provided at the distal-end portion of the inserted portion 41; and a manipulating portion 45 that has angle levers 43 and 44 for manipulating the bending portion 42 so as to bend in the up/down direction and the left/right direction, respectively. The reference sign 46 indicates a camera for acquiring endoscope images.

The manipulator 1, described below, is used in a state in which the manipulator 1 is inserted into the channel 41a of the endoscope 40.

### (First Embodiment)

Next, the manipulator 1 according to a first embodiment of the present invention will be described with reference to Figs. 2 to 5B.

As shown in Fig. 2, the manipulator 1 according to this embodiment is provided with: an elongated body portion 2 that can be inserted into the body; an end effector 3 that is positioned on the distal-end side of the body portion 2; a joint mechanism 4 that couples the body portion 2 with the end effector 3; and a driving portion 5 that is connected to the base end of the body portion 2 and that drives the joint mechanism 4.

Fig. 2 explains the overall configuration of the manipulator 1, and Figs. 3A to 5B mainly explain the joint mechanism 4. In particular, Figs. 3A to 4B explain a second joint 12, a third joint 13, and a restraining wire 7. Figs. 5A and 5B explain a first joint 11 and driving wires 9. In Figs. 3A to 5B, illustrations of some portions of the configuration are omitted.

Although the referenced drawings show forceps having a pair of gripping pieces 3a as an example of the end effector 3, the end effector 3 may be scissors, an electric scalpel, or the like.

As shown in Fig. 3A, the joint mechanism 4 is provided with, sequentially from the distal-end side: a first linkage member 61; a second linkage member 62; the first joint 11 that couples a base-end portion of the end effector 3 with a distal-end portion of the first linkage member 61 so as to be pivotable about a first flexing axis A1; the second joint 12 that couples a base-end portion of the first linkage member 61 with a distal-end portion of the second linkage member 62 so as to be pivotable about a second flexing axis A2; the third joint 13 that couples a base-end portion of the second linkage member 62 with a distal -end portion of the body portion 2 so as to be pivotable about a third flexing axis A3; a restraining wire (restraining member) 7 that extends from the first linkage member 61 to the body portion 2; a tensile-force adjusting means 8 for adjusting the tensile force in the restraining wire 7; and driving wires 9 (see Figs. 5A and 5B) that drive the first joint 11.

The first flexing axis A1 is an axis in a direction orthogonal to a longitudinal axis B of the manipulator 1. The second flexing axis A2 and the third flexing axis A3 are axes in directions orthogonal to both the longitudinal axis B and the first flexing axis A1, and are parallel to each other.

The second joint 12 and the third joint 13 are provided with a pair of guide pulleys (guide members) 21 and 22, respectively, which define paths through which the restraining wire 7 is provided. The guide pulley 21 on the distal-end side is supported by the first linkage member 61 and the second linkage member 62 so as to be rotatable about the second flexing axis A2. The other guide pulley 22 on the base-end side is supported by the second linkage member 62 and the body portion 2 so as to be rotatable about the third flexing axis A3.

The pair of guide pulleys 21 and 22 are arranged with a gap therebetween, having a width that is equal to or greater than the diameter of the restraining wire 7, in the direction of the longitudinal axis B, that is, the radial directions of the guide pulleys 21 and 22. In other words, the distance between the second flexing axis A2 and the third flexing axis A3 is equal to the sum of the radius of the guide pulley 21, the radius of the other guide pulley 22, and the diameter of the restraining wire 7 or is greater than that sum.

The restraining wire 7 is formed of one wire that does not stretch in the length direction thereof and that has a constant length while having flexibility that allows it to bend along the outer circumferential surfaces of the guide pulleys 21 and 22. As shown in Fig. 3A, the two ends of the restraining wire 7 are disposed on either side of the second flexing axis A2 so as to flank the second flexing axis A2 in the radial direction, and are individually secured to the first linkage member 61. An arbitrary method can be employed as the method for securing the two ends of the restraining wire 7 to the first linkage member 61; for example, caulking members (not shown) may be used, or end portions of the restraining wire 7 may be tied to the first linkage member 61. In the following, a portion of the restraining wire 7 that extends from one end thereof to the base end will be referred to as a first portion 71, and a portion that extends from the other end to the base end will be referred to as a second portion 72.

With the restraining wire 7, the first portion 71 and the second portion 72, forming a pair, intersect each other just once in the gap between the pair of guide pulleys 21 and 22 and are threaded around the outer circumferential surfaces of the guide pulleys 21 and 22 in a crossed manner, thus forming a single loop portion 73. In other words, as shown in Fig. 3A, in the state in which the flexing angles of the second joint 12 and the third joint 13 are 0°, the first portion 71 and the second portion 72 are threaded around the outer circumferential surfaces of the individual guide pulleys 21 and 22 by about 1/4 of the circumferences thereof. The restraining wire 7 is secured to the body portion 2 via the tensile-force adjusting means 8 at the loop portion 73.

The tensile-force adjusting means 8 includes a movable plate 8a provided in the body portion 2. The movable plate 8a is rotatable about a rotation axis C that is parallel to the third flexing axis A3. In addition, the rotation axis C is provided at a position at which the distance from the rotation axis C to the circumference of the movable plate 8a is not constant. In the illustrated example, the movable plate 8a has an elliptical shape, and the rotation axis C is provided at an eccentric position in the movable plate 8a.

The loop portion 73 of the restraining wire 7 is threaded around the outer circumferential surface of the movable plate 8a. The tensile-force adjusting means 8 is configured so that the tensile force applied to the restraining wire 7 is changed due to the changes in the pulling force of the restraining wire 7 caused by the movable plate 8a, in accordance with the rotation angle about the rotation axis C of the movable plate 8a. Therefore, during the assembly of the restraining wire 7, first, as shown in Fig. 4A, the restraining wire 7 is threaded around the outer circumferential surface of the movable plate 8a in the state in which the movable plate 8a is disposed at an angle at which the restraining wire 7 is relaxed; next, as shown in Fig. 4B, the movable plate 8a is rotated in a direction that increases the tensile force in the restraining wire 7; and the movable plate 8a is locked, in an unlockable manner, by means of a stopper (not shown) so as not to rotate about the rotation axis C. By doing so, it is possible to achieve a state in which the restraining wire 7 has a high tensile force by eliminating slack in the restraining wire 7.

By using the restraining wire 7 provided in the manner described above, the second joint 12 and the third joint 13 serve as double joints (hereinafter, also referred to as the double joints 12 and 13) that mutually work together to flex in the same direction, as shown in Fig. 3B. The reference sign 10 is a driving rod for driving the second joint 12 and the third joint 13. The driving rod 10 is disposed in the interior of the body portion 2 along the direction of the longitudinal axis B, the distal end thereof is secured to the second linkage member 62, and the base end thereof is connected to the driving portion 5.

The driving portion 5 is operated by an operator to drive individual portions of the manipulator 1, and is provided with a joystick 51 for driving the joint mechanism 4. In the case in which the end effector 3 is an electric scalpel, a switch 52 for turning on and off the supply of high-frequency current is also provided in the driving portion 5.

When the driving portion 5 moves the driving rod 10 in the direction of the longitudinal axis B in accordance with the operation input via the joystick 51, the second linkage member 62 is rotated about the third flexing axis A3, and thus, the third joint 13 is flexed. At this time, because the path lengths of the first portion 71 and the second portion 72 of the restraining wire 7 that connects the first linkage member 61 and the body portion 2 are constant, the first linkage member 61 is also rotated, so as to follow the rotation of the second linkage member 62, about the second flexing axis A2 at a rotation angle θ2 corresponding to a rotation angle θ3 about the third flexing axis A3 of the second linkage member 62. By doing so, the second joint 12 and the third joint 13 flex by working together. At this time, in particular, in the case in which the width of the gap between the pair of guide pulleys 21 and 22 is equal to the diameter of the restraining wire 7, the relationship between the flexing angle θ3 of the third joint 13 and the flexing angle θ2 of the second joint 12 is uniquely determined.

As shown in Fig. 5A, the first joint 11 is provided with a pair of drive pulleys 14 that can be rotated about the first flexing axis A1. The pair of drive pulleys 14 are provided on two sides of the first joint 11 so as to flank the longitudinal axis B therebetween, and driving wires 9 for driving the first joint 11 are threaded around the individual drive pulleys 14. In Figs. 5A and 5B, the second linkage member 62, the restraining wire 7, and the tensile-force adjusting means 8 are omitted in order to simplify the illustrations.

The driving wires 9 are secured to the drive pulleys 14 at intermediate positions in the longitudinal direction, and both ends thereof extend to the driving portion 5 by passing through the body portion 2. When the driving portion 5 pushes out one of the two ends of the driving wires 9 and pulls the other ends thereof, the drive pulleys 14 are rotated, thus changing the flexing angle of the first joint 11.

Here, the second joint 12 and the third joint 13 are provided with another pair of guide pulleys (pulleys) 31 and 32 for defining paths through which the driving wires 9 are provided. The guide pulley 31 on the distal-end side is rotatable about the second flexing axis A2, and the other guide pulley 32 on the base-end side is rotatable about the third flexing axis A3. The pair of guide pulleys 31 and 32 are arranged with a gap therebetween, having a width equal to the diameter of the driving wires 9, in the direction of the longitudinal axis B.

The two driving wires 9 extend from the drive pulleys 14 to the guide pulley 31 in the direction of the longitudinal axis B, pass through the gap between the pair of guide pulleys 31 and 32, and extend from the other guide pulley 32 to the driving portion 5 in the direction of the longitudinal axis B. By doing so, the two driving wires 9 intersect each other just once in the gap between the pair of guide pulleys 31 and 32, thus being threaded around the outer circumferential surfaces of the pair of pulleys 31 and 32 in a crossed manner.

By providing the driving wires 9 in this way, the first joint 11 and the second and third joints 12 and 13 are configured so as to be driven independently of each other. In other words, as shown in Figs. 5A and 5B, when the flexing angles θ2 and θ3 of the second and third joints 12 and 13 are changed, there is no change in the sum of the lengths of portions of the individual driving wires 9 that are in contact with the outer circumferential surfaces of the pair of guide pulleys 31 and 32, that is, the path lengths of the driving wires 9. In other words, because the driving wires 9 are not moved in the longitudinal direction, the flexing angle of the first joint 11 does not change.

Next, the operation of the thus-configured manipulator 1 according to this embodiment will be described.

With the manipulator 1 according to this embodiment, when the driving portion 5 pushes out one end of each driving wire 9 and pulls the other ends thereof, the first joint 11 is flexed, and the end effector 3 is pivoted in a direction that intersects the first flexing axis A1. In addition, when the driving portion 5 pushes out or pulls the driving rod 10, the double joints 12 and 13 are flexed, and the end effector 3 is pivoted in a direction that intersects the second and third flexing axes A2 and A3.

In this case, according to this embodiment, the restraining wire 7, which is provided between the first linkage member 61 and the body portion 2 in a crossed manner so as to flank the second flexing axis A2 and the third flexing axis A3, restrains the flexing angle θ3 of the double joints 12 and 13 with respect to the flexing angle θ2. By doing so, there is an advantage in that it is possible to make the end effector 3 pivot just by a desired angle by accurately controlling the flexing angles θ2 and θ3 of the double joints 12 and 13. In addition, the mutual relationship between the two flexing angles θ2 and θ3 of the double joints 12 and 13 is determined by the geometric arrangement of the restraining wire 7, and thus, so long as the arrangement of the restraining wire 7 is constant, the mutual relationship between the flexing angles θ2 and θ3 also becomes constant. Therefore, there is an advantage in that it is possible to achieve stable operating performance of the double joints 12 and 13.

In addition, because slippage does not occur between the guide pulleys 21 and 22, the driving wires 9 are not displaced in the length direction during flexing operation of the double joints 12 and 13, and thus, there is an advantage in that it is possible to ensure the independence of the flexing operation of the first joint 11 and the double joints 12 and 13. In addition, in the case in which the restraining wire 7 becomes slack due to repeated flexing operation of the double joints 12 and 13 or the like, the state in which the tensile force in the restraining wire 7 is high is restored again by unlocking the movable plate 8a and by increasing the tensile force in the restraining wire 7 by adjusting the rotation angle of the movable plate 8a about the rotation axis C, and thus, there is an advantage in that it is also possible to restore the independence of the flexing operation of the first joint 11 and the double joints 12 and 13. Furthermore, because fine processing and assembly of the joint mechanism 4 are not required, there is an advantage in that it is possible to suitably utilize the joint mechanism 4 in the small manipulator 1.

Note that, in this embodiment, although the restraining wire 7 is secured to the body portion 2 by threading the loop portion 73 around the movable plate 8a of the tensile-force adjusting means 8 by using one restraining wire 7, alternatively, by using two restraining wires 7 that form a pair, base ends of the individual restraining wires 7 may be secured to the body portion 2 so as to achieve a state in which the tensile forces in the restraining wires 7 are high. As the securing method, it is possible to utilize an arbitrary means, such as the above-described caulking member, tying, soldering, or the like.

### (Second Embodiment)

Next, a manipulator according to a second embodiment of the present invention will be described with reference to Figs. 6A and 6B.

The manipulator according to this embodiment differs from that of the first embodiment in that a joint mechanism 4' is provided with, instead of the restraining wire 7, sheaths (restraining members) 15 into which the driving wires 9 can be inserted. Therefore, in this embodiment, the sheaths 15 will mainly be described, and other configurations that are the same as those of the first embodiment will be given the same reference signs, and descriptions thereof will be omitted.

As shown in Figs. 6A and 6B, the sheaths 15 are cylindrical members that have flexibility and that do not stretch in the longitudinal direction. The sheaths 15 are, for example, coil sheaths in which a metal strand is tightly wound into a coil shape. The sheaths 15 are provided at two locations, that is, one each in the two driving wires 9, and accommodate portions of the lengths of the driving wires 9 so as to be movable in the longitudinal direction.

In this embodiment, the two driving wires 9 and the two sheaths 15 provided on the individual driving wires 9 are threaded around the common pair of guide pulleys 21 and 22 in a crossed manner, as with the driving wires 9 and the restraining wire 7 in the first embodiment. However, the width of the gap between the pair of guide pulleys 21 and 22 is equal to the diameter of the sheaths 15 or is greater than the diameters of the sheaths 15. In other words, the guide pulleys 21 and 22 for the sheaths 15 also serve as the above-described guide pulleys 31 and 32 for the driving wires 9.

The individual sheaths 15 are secured to the first linkage member 61 at the distal ends and are secured to the body portion 2 at the base ends so that there is no slack. An arbitrary method, for example, soldering or the like, can be employed as the method of securing the sheaths 15 to the first linkage member 61 and the body portion 2.

With this embodiment, by integrally providing the sheaths 15, which serve as the restraining members, and the driving wires 9, there is an advantage in that the assembly of the joint mechanism 4' is facilitated, and also that it is possible to prevent the driving wires 9 from derailing from the guide pulleys 21 and 22. In addition, because the sheaths 15 are disposed coaxially with the driving wires 9, there is only a slight increase in size for providing the sheaths 15, and thus, there is an advantage in that it is possible to suitably utilize the sheaths 15 also in a small manipulator. Because other effects of this embodiment are the same as those of the first embodiment, descriptions thereof will be omitted.

Note that, in this embodiment, although the two ends of the sheaths 15 are respectively secured to the first linkage member 61 and the body portion 2, alternatively, the ends may be left unsecured. Even if the two ends of the sheaths 15 are not secured, because the flexing angle θ2 of the second joint 12 with respect to the flexing angle θ3 of the third joint 13 is restrained by the sheaths 15 having constant lengths, it is possible to make these two joints 12 and 13 work together.

In addition, in the first and second embodiments, although paths through which the restraining wire 7 is provided are defined by the pair of guide pulleys 21 and 22, alternatively, other guide members may be used.

For example, as shown in Figs. 7A and 7B, guide members 21' and 22' may be integrally provided at the distal end of the body portion 2 and the base end of the first linkage member 61, respectively, and may be semicircular members having substantially arc-like outer circumferential surfaces centered on the second flexing axis A2 and the third flexing axis A3, respectively. Fig. 7A shows a state in which the driving wires 9 and the sheaths 15 are omitted, and Fig. 7B shows a state in which the driving wires 9 and the sheaths 15 are attached to the guide members 21' and 22'. In this way, by integrally providing the guide member 21' and the first linkage member 61, which are on the distal-end side, and by integrally providing the guide member 22' and the body portion 2, which are on the base-end side, it is possible to achieve a cost reduction by reducing the number of components and the number of assembly steps. Note that, although Figs. 7A and 7B show an example configuration provided with the sheaths 15 of the second embodiment, it is possible to apply the guide members 21' and 22' to a configuration provided with the restraining wire 7 of the first embodiment.

In addition, in the first and second embodiments, although the configuration in which the double joints 12 and 13 are driven by using the driving rod 10 has been described, a driving wire 10' connected to the second linkage member 62 may be used instead of the driving rod 10, as shown in Fig. 8. The driving wire 10' is secured to a second linkage member 62' at an intermediate position in the longitudinal direction, and, by being pushed and pulled by the driving portion 5 at the two ends thereof, the driving wire 10' can rotate the second linkage member 62' about the third flexing axis A3, as with the above-described driving wires 9. The driving wire 10' has greater flexibility than does the driving rod 10. Therefore, in the case in which high flexibility is required for the body portion 2, it is preferable that the driving wire 10' be employed rather than the driving rod 10.

### {Reference Signs List}

- 1: manipulator
- 2: body portion
- 3: end effector
- 3a: gripping piece
- 4, 4': joint mechanism
- 5: driving portion
- 61: first linkage member
- 62: second linkage member
- 7: restraining wire (restraining member)
- 8: tensile-force adjusting means
- 8a: movable plate
- 9: driving wire
- 10: driving rod
- 10': driving wire
- 11: first joint
- 12: second joint
- 13: third joint
- 14: drive pulley
- 15: sheath (restraining member)
- 21, 22: guide pulley (guide member)
- 21', 22': guide member
- 31, 32: guide pulley (pulley)
- A1: first flexing axis
- A2: second flexing axis
- A3: third flexing axis
- B: longitudinal axis of body portion

## Claims

1. A manipulator comprising:
an elongated body portion;
an end effector that is positioned on a distal-end side of the body portion; and
a joint mechanism that couples the body portion with the end effector so as to be mutually pivotable about a flexing axis that intersects a longitudinal axis of the body portion,
wherein the joint mechanism is provided with:
a first linkage member and a second linkage member, sequentially from the distal-end side;
a first joint that couples the end effector with the first linkage member so as to be mutually pivotable about a first flexing axis that intersects the longitudinal axis;
a second joint that couples the first linkage member with the second linkage member so as to be mutually pivotable about a second flexing axis that intersects the longitudinal axis;
a third joint that couples the second linkage member with the body portion so as to be mutually pivotable about a third flexing axis that is parallel to the second flexing axis; and
a pair of restraining members that extend from the first linkage member to the body portion by bridging the second joint and the third joint and that have constant lengths, and
wherein the pair of restraining members flank the second flexing axis and the third flexing axis in radial directions, respectively, and are disposed in a crossed manner by intersecting each other just once between the second joint and the third joint.

2. A manipulator according to Claim 1, further comprising:
a driving wire that extends from the first joint to the body portion and that drives the first joint by being pushed and pulled in longitudinal directions thereof; and
a pair of pulleys that are provided in the second joint and the third joint so as to be individually rotatable about the second flexing axis and the third flexing axis, and that are arranged with a gap therebetween, having a size equal to or greater than the diameter of the driving wire, in a radial direction,
wherein the driving wire passes through the gap between the pair of pulleys and is threaded around outer circumferential surfaces of the pair of pulleys in a crossed manner.

3. A manipulator according to Claim 2, wherein the pair of restraining members are cylindrical sheaths into which the driving wire is inserted in a longitudinal direction in a movable manner.

4. A manipulator according to any one of Claims 1 to 3, wherein the pair of restraining members are secured to the first linkage member at the distal ends thereof and are secured to the body portion at the base ends thereof.

5. A manipulator according to any one of Claims 1 to 4, wherein the pair of restraining members have the same size in the length direction thereof.

6. A manipulator according to Claim 1 or 2,
wherein the pair of restraining members are connected with each other at the body portion, and
the manipulator further comprising:
a tensile-force adjusting means that is provided in the body portion, that imparts tensile forces to the restraining members, and that is capable of adjusting the magnitudes of the tensile forces.

7. A manipulator according to any one of Claims 1 to 6, further comprising:
a pair of guide members that are centered on the second flexing axis and the third flexing axis, respectively, and that have substantially arc-like outer circumferential surfaces that face each other in the direction of the longitudinal axis,
wherein a gap, having a width that is equal to or greater than the diameter of the restraining members, is provided between the outer circumferential surfaces of the pair of guide members, and
the restraining members pass through the gap and are threaded, in a crossed manner, around the substantially arc-like outer circumferential surfaces of the pair of guide members.

8. A manipulator according to Claim 7, wherein the width of the gap is equal to the diameter of the restraining members.

9. A manipulator according to Claim 7 or 8, wherein, of the pair of guide members, the one on a distal-end side is integrally provided with the first linkage member at a base end of the first linkage member, and the other one on a base-end side is integrally provided with the body portion at a distal end of the body portion.
